# EUROPÄISCHE PATENTSCHRIFT

(11) **EP 3 775 369 B1**
(45) Veröffentlichungstag und Bekanntmachung des Hinweises auf die Patenterteilung: **06.04.2022**
(21) Anmeldenummer: 19722899.2
(22) Anmeldetag: 08.05.2019
(51) Int. Cl.: D21F 5/00

(54) **VERFAHREN UND VORRICHTUNG ZUM TROCKNEN VON VORZUGSWEISE EINER LAUFENDEN MATERIALBAHN MIT WENIGSTENS EINER MIT BIOGAS BEHEIZTEN UND INFRAROT-STRAHLER UMFASSENDEN TROCKNUNGSEINRICHTUNG**
METHOD AND APPARATUS FOR DRYING A PREFERABLY TRAVELING MATERIAL WEB BY MEANS OF AT LEAST ONE DRYING DEVICE HEATED BY MEANS OF BIOGAS AND COMPRISING INFRARED RADIATORS
PROCÉDÉ ET DISPOSITIF DE SÉCHAGE DE PRÉFÉRENCE D'UNE BANDE DE MATÉRIAU EN MOUVEMENT, POURVU D'AU MOINS UN SYSTÈME DE SÉCHAGE CHAUFFÉ AU BIOGAZ ET COMPRENANT DES ÉMETTEURS DE RAYONS INFRAROUGES

(30) Priorität: 16.05.2018 DE 102018003969
(43) Veröffentlichungstag der Anmeldung: 17.02.2021
(73) Patentinhaber: MERI Environmental Solutions GmbH, 81243 München (DE); Voith Patent GmbH, 89522 Heidenheim (DE)
(72) Erfinder: MENKE, Lucas, 81545 München (DE); TROUBOUNIS, George, 80331 München (DE); LAUBROCK, Henning, 81673 München (DE); KÜCKMANN, Philipp, 41238 Mönchengladbach (DE)
(74) Vertreter: Henkel & Partner mbB
(86) Internationale Anmeldenummer: PCT/EP2019/061853
(87) Internationale Veröffentlichungsnummer: WO 2019/219484

(56) Entgegenhaltungen:
- EP-A1- 3 296 556
- EP-A2- 1 295 987
- WO-A1-99/51532
- DE-A1- 10 340 074
- DE-A1-102010 062 198
- US-A- 5 966 835

## Beschreibung

Die vorliegende Erfindung betrifft ein Verfahren zum Herstellen eines Gegenstandes, wie insbesondere einer laufenden Materialbahn, wie einer gestrichenen Papier- oder Kartonbahn, wobei das Verfahren das Trocknen des Gegenstandes oder eines Vorprodukts davon mit wenigstens einer Trocknungseinrichtung, welche einen oder mehrere Infrarot-Strahler aufweist, umfasst. Zudem betrifft die vorliegende Erfindung eine insbesondere zur Durchführung des Verfahrens geeignete Vorrichtung.

Es gibt eine Vielzahl von Verfahren, insbesondere kontinuierlichen Verfahren, zum Herstellen eines Gegenstandes, bei dem der Gegenstand oder ein Vorprodukt davon mit einer oder mehreren Trocknungseinrichtungen, wie beispielsweise mit einem oder mehreren Infrarot-Strahlern, getrocknet wird. Ein Beispiel für solche Verfahren ist die Herstellung von Materialbahnen, wie Papier-, Karton- oder Tissuebahnen und insbesondere von gestrichenen Papier- oder Kartonbahnen. Gestrichenes Papier ist ein Papier, dessen Oberfläche mit einem Bindemittelauftrag bzw. Strich veredelt ist, beispielsweise um dem Papier eine geschlossenere, glattere und/oder stabilere Oberfläche zu verleihen, um zum Beispiel die Detailwiedergabemöglichkeit zu erhöhen und beim Druck eine bessere Qualität zu erreichen. Dabei wird der Strich als dünner flüssiger Film auf das Papier aufgebracht, bevor das so beschichtete Papier dann später getrocknet wird. Beispielsweise offenbart die EP 1 295 987 A2 ein solches Verfahren zum Trocknen einer gestrichenen Papier- oder Kartonbahn, bei dem die Bahn zunächst in einem Infrarot-Trockner mit Infrarot-Strahlern vorgetrocknet und anschließend in einem Lufttrockner mit Luft weiter getrocknet wird, wobei der Lufttrockner so betrieben wird, dass der Wärmeübergangskoeffizient zwischen der Trocknungsluft und der Bahn in Bahnlaufrichtung ansteigend verläuft.

Üblicherweise werden die Infrarot-Strahler elektrisch oder mit Erdgas beheizt. Allerdings sind die Betriebskosten insbesondere für elektrisch beheizte Infrarot-Strahler sehr hoch. Aus diesem Grund werden die Infrarot-Strahler in entsprechenden großtechnischen Verfahren vorwiegend mit Erdgas beheizt. Gleiches gilt auch für Lufttrockner, die häufig mit Erdgas als Primärenergiequelle oder mit einer Sekundärquelle, wie z.B. Dampf, versorgt werden. Jedoch sind die Betriebskosten auch für beide Arten der Trocknung sehr hoch und betragen beispielsweise bei den Verfahren zur Herstellung von gestrichenem Papier 15 bis 20% der gesamten Betriebskosten.

Die DE 103 40 074 A1 offenbart eine Anlage zur Verbrennung von schwach methanhaltigen Gasen, wie Deponiegas, bei dem das schwach methanhaltige Gas über einen Oxidationskörper geströmt wird und dessen Verbrennung aufgrund der Prozesswärme eingeleitet wird. Dabei kann die Steuerung des Verbrennungsprozesses über die gesteuerte Luftzufuhr erfolgen, welche durch eine Restsauerstoffgehaltsmessung im Abgas geregelt wird.

In der DE 10 2010 062198 A1 wird ein Verfahren zum Betrieb eines Otto-Gasmotors vorzugsweise mit Brenngas in Form von Biogas beschrieben, bei dem der Zündzeitpunkt des Verbrennungs-Luftgemisches eingestellt wird, und zwar in Abhängigkeit von der gemessenen Abgastemperatur und der Drossel-Stellung.

Aus der EP 3 296 556 A1 ist ein nur mit Biogas betriebener Motor bekannt, bei zur Einstellung eines optimalen Luft-Treibstoff-Verhältnisses ein Venturi-Mischer vorgesehen ist, durch den das Biogas mit einer optimalen Strömungsgeschwindigkeit angesaugt wird.

Aufgabe der vorliegenden Erfindung ist es daher, ein Verfahren zum Herstellen eines Gegenstandes, wie insbesondere einer laufenden Materialbahn, wie einer gestrichenen Papier- oder Kartonbahn, bereitzustellen, welches das Trocknen des Gegenstandes oder eines Vorprodukts davon mit wenigstens einer Trocknungseinrichtung mit wenigstens einem Infrarot-Strahler umfasst, bei dem die Betriebskosten und insbesondere die auf das Trocknen mit der wenigstens einen Trocknungseinrichtung entfallenen Betriebskosten erheblich verringert sind, aber dennoch ein vorgegebener Trocknungsgrad des Gegenstandes präzise erreicht wird.

Erfindungsgemäß wird diese Aufgabe gelöst durch ein Verfahren zum Herstellen eines Gegenstandes, wie insbesondere einer laufenden Materialbahn, wie einer gestrichenen Papier- oder Kartonbahn, wobei das Verfahren das Trocknen des Gegenstandes oder eines Vorprodukts davon umfasst, bei dem der Gegenstand oder ein Vorprodukt davon in wenigstens einer Trocknungseinrichtung zumindest teilweise getrocknet wird, wobei mindestens eine der wenigstens einen Trocknungseinrichtung wenigstens einen zumindest teilweise mit Biogas beheizten Infrarot-Strahler enthält, dem Biogas vor dessen Zuführung in den wenigstens einen Infrarot-Strahler zur Beheizung desselben ein aus der aus Sauerstoff, Luft oder anderen Sauerstoff enthaltenden Gasen bestehenden Gruppe ausgewähltes Verbrennungsgas zugemischt wird und in dem durch das Beheizen des wenigstens einen Infrarot-Strahlers mit dem Biogas anfallenden Abgas mit einer Messeinrichtung der Sauerstoffgehalt gemessen wird und aufgrund des gemessenen Sauerstoffgehalts die Menge an dem Biogas vor dessen Zuführung in den wenigstens einen Infrarot-Strahler zugemischten Verbrennungsgas geregelt wird.

Indem die für den Trocknungsvorgang in der Trocknungseinrichtung eingesetzten ein oder mehreren Infrarot-Strahler weder elektrisch noch mit Erdgas beheizt werden, sondern mit Biogas, können die auf das Trocknen mit der Trocknungseinrichtung entfallenen Betriebskosten verringert werden, weil Biogas, wie insbesondere Methan enthaltendes Biogas, billiger als Erdgas zu beziehen ist, und zwar insbesondere in dem Fall, dass das Biogas nahe der Trocknungseinrichtung produziert wird, und ganz besonders in dem Fall, dass das Biogas in derselben Anlage aus Abfallprodukten des Verfahrens produziert wird, da dann die Kosten für die Abfallentsorgung zumindest teilweise eingespart werden können. Diese Kostenvorteile werden in besonders hohem Ausmaß erreicht, wenn alle der eingesetzten Trocknungseinrichtungen jeweils ein oder mehrere Infrarot-Strahler aufweisen und alle der Infrarot-Strahler vollständig mit Biogas, welches vorzugsweise Methan enthält, beheizt werden. Unter Biogas wird in diesem Zusammenhang ein brennbares Gas verstanden, das durch Verarbeiten von Biomasse jeder Art erzeugt wird, wie beispielsweise durch Mikroorganismen, welche Biomasse, wie organische Verunreinigungen, zu Methan oder anderen brennbaren Substanzen umsetzen.

Wie dargelegt ergibt sich bei dem erfindungsgemäßen Verfahren insbesondere dann eine erhebliche Kosteneinsparung gegenüber den aus dem Stand der Technik bekannten, wenn das zur Beheizung der Trocknungseinrichtung eingesetzte Biogas in derselben Anlage hergestellt wird, in welcher das Verfahren zum Herstellen und Trocknen des Gegenstandes durchgeführt wird, und zwar bevorzugt aus in dem Verfahren selbst erzeugten Abfallprodukten. Zum einen kann dadurch auf die Infrastruktur zur Zuführung von Erdgas verzichtet werden und zudem kann auf die kostenträchtige Entsorgung der erzeugten Abfallprodukte vollständig oder zumindest teilweise verzichtet werden, indem die Abfallprodukte zur Erzeugung des Biogases genutzt werden.

Kennzeichnend für die eingesetzte Trocknungseinheit ist, dass diese einen Energie- und Stoffübergang in dem zu trocknenden Gut herbeiführt. Die Energieübertragung kann dabei sowohl durch Kontakt, Konvektion und/oder elektromagnetische Strahlung erfolgen, wobei die flüssige Phase des zu trocknenden Gutes teilweise seinen Aggregatszustand verändert und das Gut verlässt. Dabei kann das Biogas zum Beheizen der Trocknungseinheit als Primärenergiequelle eingesetzt werden, aber alternativ dazu auch über den weiteren Schritt eines Sekundärenergiestromes, wie z.B. Dampf.

Erfindungsgemäß ist es vorgesehen, dass eine oder mehrere der zumindest teilweise mit Biogas beheizten Trocknungseinrichtungen jeweils ein oder mehrere Infrarot-Strahler enthalten, wobei wenigstens einer und bevorzugt alle der Infrarot-Strahler zumindest teilweise mit Biogas beheizt werden. Vorzugsweise weist jede Trocknungseinrichtung ein oder mehrere Infrarot-Strahler, wobei alle Infrarot-Strahler vollständig mit Biogas beheizt werden.

Gemäß einer bevorzugten Ausführungsform der vorliegenden Erfindung wird es vorgeschlagen, dass bei dem Verfahren auch Abwasser und/oder ein anderer Reststoff erzeugt wird, wobei das Biogas, welches vorzugsweise Methan enthält, durch Behandeln des erzeugten Abwassers und/oder anderen Reststoffs hergestellt wird. Dies lässt sich beispielsweise leicht bei der Herstellung von Papier-, Karton- oder Tissuebahnen, wie beispielsweise von gestrichenen Papier- oder Kartonbahnen, erreichen, da bei diesen Verfahren erhebliche Mengen an organisch belastetem Abwasser anfallen, das in einem Großteil der bestehenden Anlagen mit anaerobe Mikroorganismen enthaltenden Reaktoren gereinigt wird. Bei der Aufreinigung mit anaeroben Mikroorganismen werden die organischen Verunreinigungen zu einem Methan und Kohlendioxid enthaltendem Biogas umgesetzt, welches bei den bestehenden Anlagen größtenteils ungenutzt entsorgt wird. Gemäß der vorliegenden Erfindung kann dieses Biogas zur Beheizung der zur Trocknung der Papier-, Karton- oder Tissuebahnen in der Trocknungseinrichtung eingesetzten Infrarot-Strahler eingesetzt werden. Gleiches lässt sich ebenfalls beispielsweise bei der Herstellung von Kartoffelchips realisieren, bei der mit den anfallenden Kartoffelschalen eine erhebliche Menge an Biomasseabfall anfällt, der leicht über Vergärung zu Methan enthaltenem Biogas umgesetzt werden kann.

Alternativ dazu oder auch zusätzlich dazu, kann es zu demselben Zweck vorgesehen sein, dass bei dem Verfahren Prozesswasser im Kreislauf geführt wird, welches bei dem Verfahren beispielsweise mit organischen Verbindungen verunreinigt wird, wobei das Biogas, insbesondere Methan enthaltendes Biogas, durch Behandeln des erzeugten Prozesswassers hergestellt wird. Auch dies lässt sich leicht bei der Herstellung von Papier-, Karton- oder Tissuebahnen, wie beispielsweise von gestrichenen Papier- oder Kartonbahnen, erreichen, da bei diesen Verfahren erhebliche Mengen an Prozesswasser im Kreislauf geführt werden, wobei das Prozesswasser bei dem Verfahren mit organischen Verbindungen verunreinigt wird. Dieses Prozesswasser muss in den bestehenden Anlagen gereinigt werden und wird im großen Umfang bereits mit anaerobe Mikroorganismen enthaltenden Reaktoren gereinigt. Dabei werden, wie dargelegt, die organischen Verunreinigungen zu einem Methan und Kohlendioxid enthaltendem Biogas umgesetzt, welches bei den bestehenden Anlagen größtenteils ungenutzt entsorgt wird.

Aus den vorgenannten Gründen ist es gemäß der vorliegenden Erfindung daher insbesondere bevorzugt, dass bei dem Verfahren Abwasser erzeugt wird und/oder Prozesswasser im Kreislauf geführt wird, welches bei dem Verfahren beispielsweise mit organischen Verbindungen verunreinigt wird, wobei das Behandeln des Abwassers und/oder Prozesswassers in einem anaerobe Mikroorganismen enthaltendem Reaktor durchgeführt wird, wobei die anaeroben Mikroorganismen in dem Abwasser und/oder Prozesswasser enthaltende organische Verbindungen zu einem Methan enthaltendem Biogas umsetzen. Wie dargelegt lässt sich diese Ausführungsform insbesondere leicht bei der Herstellung von Papier-, Karton- oder Tissuebahnen, wie beispielsweise von gestrichenen Papier- oder Kartonbahnen, realisieren.

Als mit anaeroben Mikroorganismen versetzter anaerober Reaktor können dabei alle dem Fachmann bekannten Typen von anaeroben Reaktoren eingesetzt werden, beispielsweise Kontaktschlammreaktoren, UASB-Reaktoren, EGSB-Reaktoren, Festbettreaktoren und Fließbettreaktoren, wobei insbesondere mit UASB-Reaktoren und EGSB-Reaktoren gute Ergebnisse erzielt werden. Bei den UASB- und EGSB-Reaktoren wird üblicherweise dem Reaktor über einen Zulauf im unteren Reaktorbereich kontinuierlich zu reinigendes Abwasser zugeführt und dieses durch ein oberhalb des Zulaufs befindliches, Mikroorganismenpellets enthaltendes Schlammbett geführt. Bei dem Abbau der organischen Verbindungen aus dem Abwasser bilden die Mikroorganismen insbesondere Methan und Kohlendioxid enthaltendes Biogas, das sich teilweise in Form kleiner Bläschen an den Mikroorganismenpellets anlagert und teilweise in Form freier Gasbläschen in dem Reaktor nach oben steigt. Aufgrund der angelagerten Gasbläschen sinkt das spezifische Gewicht der Pellets, weshalb die Pellets in dem Reaktor nach oben steigen. Um das gebildete Biogas und die aufsteigenden Pellets von dem Wasser zu trennen, sind in dem mittleren und/oder oberen Teil des Reaktors typischerweise Abscheider zumeist in Form von Gashauben angeordnet, unter deren First sich Biogas ansammelt, welches ein Gaspolster ausbildet, worunter eine Flotationsschicht aus Mikroorganismenpellets und Abwasser befindlich ist. Von Gas und Mikroorganismenpellets befreites, gereinigtes Wasser steigt in dem Reaktor nach oben und wird am oberen Ende des Reaktors über Überläufe abgezogen, wohingegen die von den Gasbläschen befreiten Mikroorganismenpellets aufgrund des nunmehr erhöhten spezifischen Gewichts in dem Reaktor wieder nach unten absinken. Derartige Verfahren und entsprechende Reaktoren sind beispielsweise in der EP 0 170 332 A1, in der EP 1 071 636 B1 und in der EP 0 539 430 B1 beschrieben.

Gemäß einer besonders bevorzugten Ausführungsform der vorliegenden Erfindung wird mit dem erfindungsgemäßen Verfahren eine laufende Materialbahn, vorzugsweise eine gestrichene Papier- oder Kartonbahn, hergestellt und diese in der wenigstens einen Trocknungseinrichtung zumindest teilweise getrocknet, wobei bei dem Verfahren organische Verbindungen enthaltendes Abwasser anfällt und/oder Prozesswasser im Kreislauf geführt wird, welches bei dem Verfahren mit organischen Verbindungen verunreinigt wird, wobei das Biogas durch Behandeln des Abwassers und/oder Prozesswassers in einem anaerobe Mikroorganismen enthaltendem Reaktor erzeugt wird, wobei die anaeroben Mikroorganismen in dem Abwasser und/oder Prozesswasser enthaltende organische Verbindungen zu dem Biogas umsetzen.

Alternativ dazu kann das erfindungsgemäße Verfahren auch so eingesetzt werden, dass bei dem Verfahren ein anderer Reststoff als Abwasser oder verunreinigtes Prozesswasser erzeugt wird, wobei das Biogas durch Vergären des erzeugten anderen Reststoffs hergestellt wird. Diese Ausführungsform ist beispielsweise für Verfahren zur Herstellung von Kartoffelchips geeignet, bei denen die frittierten Kartoffelchips in einer Infrarot-Strahler umfassenden Trocknungseinrichtung getrocknet werden. Bei diesem Verfahren fallen erhebliche Menge an Biomasseabfall in Form von Kartoffelschalen an, die leicht über Vergärung zu Methan enthaltenem Biogas umgesetzt werden können.

Wie dargelegt, enthält das eingesetzte Biogas vorzugsweise Methan, weil Methan einen hohen Heizwert aufweist und als Stoffwechselendprodukt durch anaerobe Mikroorganismen leicht hergestellt werden kann. Vorzugsweise enthält das Biogas, welches in dem Verfahren, wie dargelegt, besonders bevorzugt selbst hergestellt wird, bezogen auf 100 Gew-%, mindestens 50 Gew.-%, weiter bevorzugt mindestens 60 Gew.-% und ganz besonders bevorzugt mindestens 70 Gew.-% Methan.

Um eine gute Verbrennung des Biogases zu gewährleisten und ggf. störende Bestandteile zu entfernen, wird es in Weiterbildung des Erfindungsgedankens vorgeschlagen, das Biogas vor dessen Verwendung zum Beheizen des wenigstens einen Infrarot-Strahlers zu konditionieren, wobei das Biogas zur Konditionierung bevorzugt einem oder mehreren der folgenden Behandlungsschritte unterzogen wird:
i) Gastrocknung zum Entfeuchten des Biogases, um die Verbrennbarkeit zu erhöhen,
ii) Kompression zum Verdichten des Biogases, so dass dieses einen geeigneten Heizwert pro Volumeneinheit aufweist,
iii) Entschwefelung zur Entfernung von störenden schwefelhaltigen Verbindungen, wie insbesondere von Schwefelwasserstoff,
iv) Reinigung mit Aktivkohle zur Entfernung von störenden Verbindungen, wie insbesondere von Schwefelwasserstoff.

Gemäß einer weiteren bevorzugten Ausführungsform ist es vorgesehen, dass das Biogas vor dessen Verwendung zum Beheizen des wenigstens einen Infrarot-Strahlers in einem Puffertank zwischengespeichert wird. Dies ist deshalb vorteilhaft, weil Biogas über den Herstellungszeitraum Änderungen in der Zusammensetzung unterliegen kann, wie insbesondere des Methangehalts und der produzierten Menge. Beispielsweise schwankt der Verunreinigungsgrad des bei der Papierherstellung anfallenden verunreinigten Abwassers und Prozesswassers über die Zeit, weswegen auch das bei der Reinigung des Abwassers und Prozesswassers durch anaerobe Mikroorganismen erzeugte Biogas bezüglich seiner Menge und/oder seiner Zusammensetzung, wie beispielsweise des Methangehalts, zeitlichen Änderungen unterliegt. Wenn der Methangehalt des Biogases sinkt, muss mehr Biogas pro Zeiteinheit zur Beheizung der Infrarotstrahler eingesetzt werden, wohingegen die Menge an pro Zeiteinheit zur Beheizung der Trocknungseinrichtung, nämlich der Infrarotstrahler, benötigtem Biogas entsprechend geringer ist, wenn sich der Methangehalt des Biogases erhöht. Indem das Biogas vor dessen Verwendung zum Beheizen des wenigstens einen Infrarot-Strahlers in einem Puffertank zwischengespeichert wird, kann diesen Schwankungen der Zusammensetzung des Biogases und/oder Schwankungen der pro Zeiteinheit erzeugten Menge des Biogases Rechnung getragen werden, um eine kontinuierliche Zuführung einer ausreichenden Menge des Biogases zu dem wenigstens einen Infrarot-Strahler auch bei Schwankungen der Zusammensetzung des Biogases und/oder Schwankungen der erzeugten Menge des Biogases zu gewährleisten.

Um eine gute Verbrennung und somit gute Ausnutzung des Heizwertes des Biogases zu erreichen, wird dem Biogas erfindungsgemäß vor dessen Zuführung in den wenigstens einen Infrarot-Strahler zur Beheizung desselben vorzugsweise ein aus der aus Sauerstoff, Luft oder anderen Sauerstoff enthaltenden Gasen bestehenden Gruppe ausgewähltes Verbrennungsgas zugemischt.

Um eine optimale Verbrennung in dem wenigstens einen Infrarot-Strahler und somit Ausnutzung des Heizwertes des Biogases zu erreichen, ist es erfindungsgemäß vorgesehen, den Sauerstoffgehalt in dem durch das Beheizen des wenigstens einen Infrarot-Strahlers mit dem Biogas anfallenden Abgas mit einem Sensor zu messen und aufgrund des gemessenen Sauerstoffgehalts die Menge an dem Biogas vor dessen Zuführung in den wenigstens einen Infrarot-Strahler zugemischten Verbrennungsgas zu regeln. Diese Messung des Sauerstoffgehalts in dem durch das Beheizen des wenigstens einen Infrarot-Strahlers mit dem Biogas durch Verbrennen des Biogases mit dem Verbrennungsgas in dem wenigstens einen Infrarot-Strahler anfallenden Abgas wird mit einer Messeinrichtung, wie beispielsweise mit einem Sensor, durchgeführt. Besonders bevorzugt wird als Messeinrichtung eine Lambdasonde eingesetzt. Vorzugsweise ist die Messeinrichtung bzw. Lambdasonde direkt vor dem wenigstens einen Infrarot-Strahler angeordnet, um so ein optimales Gemisch aus Biogas und Verbrennungsgas einzustellen, damit sowohl ein Überangebot an kühlender Zuluft als auch infolge von Sauerstoffmangel entstehendes Kohlenmonoxid mit ungenutztem Restheizwert in dem wenigstens einen Infrarot-Strahler verhindert wird.

In Weiterbildung des Erfindungsgedankens wird es vorgeschlagen, den Heizwert des Biogases vor dessen Zuführung in den wenigstens einen Infrarot-Strahler zu bestimmen. Dies kann auf alle dem Fachmann bekannten Arten erfolgen. Beispielsweise kann zunächst der Brennwert mit einem Verbrennungskalorimeter bestimmt werden, bevor der Heizwert aus dem Brennwert bekanntermaßen durch Abziehen der Verdampfungsenthalpie des Wassers von dem Brennwert berechnet wird.

Vorzugsweise wird aufgrund des bestimmten Heizwertes des Biogases die Menge des pro Zeiteinheit dem wenigstens einen Infrarot-Strahler zuzuführenden Biogases geregelt.

Insbesondere ist es bevorzugt, die Menge des pro Zeiteinheit dem wenigstens einen Infrarot-Strahler zuzuführenden Biogases sowie die Menge des pro Zeiteinheit dem Biogas zuzuführenden Verbrennungsgases so zu regeln, dass der wenigstens eine Infrarot-Strahler eine gleichbleibende Wärmeabstrahlleistung aufweist.

Beispielsweise in dem Fall, dass aus produktionstechnischen Gründen zeitweise die Menge an in dem Verfahren erzeugten Biogas für eine ausreichende Beheizung der Infrarot-Strahler zu gering ist, kann es zweckmäßig sein, dem Biogas vor dessen Zuführung in den wenigstens einen Infrarot-Strahler zur Beheizung desselben (geringe Mengen an) Erdgas zuzumischen.

Ferner kann es in dem Fall, dass aus produktionstechnischen Gründen zeitweise der Heizwert bzw. Methangehalt des in dem Verfahren erzeugten Biogases für eine ausreichende Beheizung der Infrarot-Strahler zu gering ist, nötig sein, dem Biogas vor dessen Zuführung in den wenigstens einen Infrarot-Strahler zur Beheizung desselben Methan zuzumischen.

Ein weiterer Gegenstand der vorliegenden Erfindung ist eine Vorrichtung zum Herstellen eines Gegenstandes, wie insbesondere einer laufenden Materialbahn, wie einer gestrichenen Papier- oder Kartonbahn, welches insbesondere zur Durchführung des zuvor beschriebenen Verfahrens geeignet ist, wobei die Vorrichtung umfasst:
a) wenigstens eine Trocknungseinrichtung, wobei wenigstens eine Trocknungseinrichtung ein oder mehrere Infrarot-Strahler enthält,
b) eine Vorrichtung zur Erzeugung von Biogas aus wenigstens einem ausgewählt aus der Gruppe bestehend aus Abwasser, Prozesswasser, anderen Reststoffen und beliebigen Kombinationen von zwei oder mehr der vorgenannten,
c) eine Leitung zur Zuführung von Biogas aus der Vorrichtung zur Erzeugung von Biogas zu wenigstens einer der wenigstens einen Trocknungseinrichtung und
d) wenigstens eine Verbrennungsgaszufuhrleitung zur Zumischung von einem aus der aus Sauerstoff, Luft oder anderen Sauerstoff enthaltenden Gasen bestehenden Gruppe ausgewählten Verbrennungsgas zu dem Biogas vor dessen Zuführung in die wenigstens eine Trocknungseinrichtung, wobei die Vorrichtung außerdem eine Abgasleitung zur Abführung des in dem wenigstens einen Infrarot-Strahler erzeugten Verbrennungsabgases aus dem wenigstens einen Infrarot-Strahler umfasst, wobei in der Abgasleitung eine Messeinrichtung angeordnet ist, die dazu ausgelegt ist, den Sauerstoffgehalt in dem durch das Beheizen des wenigstens einen Infrarot-Strahlers mit dem Biogas anfallenden Abgases zu messen, die Vorrichtung des Weiteren eine Regeleinheit umfasst, welche aufgrund des durch die Messeinrichtung gemessenen Sauerstoffgehalts die Menge an dem Biogas vor dessen Zuführung in den wenigstens einen Infrarot-Strahler über die Zufuhrleitung zuzuführenden und zuzumischenden Verbrennungsgas regelt, und die Leitung zur Zuführung von Biogas aus der Vorrichtung zur Erzeugung von Biogas zu wenigstens einem Infrarot-Strahler ausgelegt ist.

Erfindungsgemäß enthält die wenigstens eine Trocknungseinrichtung ein oder mehrere Infrarot-Strahler und ist die Leitung zur Zuführung von Biogas aus der Vorrichtung zur Erzeugung von Biogas zu wenigstens einem Infrarot-Strahler ausgelegt, d. h. die Leitung zur Zuführung von Biogas aus der Vorrichtung zur Erzeugung von Biogas führt zu bzw. in wenigstens einen Infrarot-Strahler.

Vorzugsweise ist die Vorrichtung zur Erzeugung von Biogas ein Reaktor, der anaerobe Mikroorganismen enthält, welche organische Verbindungen zu einem Methan enthaltendem Biogas umsetzen können.

Gemäß einer weiteren bevorzugten Ausführungsform der vorliegenden Erfindung umfasst die Vorrichtung ferner wenigstens eine der folgenden Konditionierungseinrichtungen:
i) einen oder mehrere Gastrockner zum Entfeuchten des Biogases,
ii) einen oder mehrere Kompressoren zum Verdichten des Biogases,
iii) eine oder mehrere Entschwefelungseinrichtungen zur Entfernung von schwefelhaltigen Verbindungen,
iv) eine oder mehrere Aktivkohle enthaltende Reinigungseinrichtungen.

Ferner ist es bevorzugt, dass die Vorrichtung zudem einen in der Leitung angeordneten Puffertank umfasst, in dem Biogas vor dessen Verwendung zum Beheizen des wenigstens einen Infrarot-Strahlers zwischengespeichert wird, um eine kontinuierliche Zuführung einer ausreichenden Menge des Biogases zu dem wenigstens einen Infrarot-Strahler auch bei Schwankungen der Zusammensetzung des Biogases und/oder Schwankungen der erzeugten Menge des Biogases gewährleisten zu können.

Erfindungsgemäß ist es vorgesehen, dass die Vorrichtung außerdem eine Abgasleitung zur Abführung des in dem wenigstens einen Infrarot-Strahler erzeugten Verbrennungsabgases aus dem wenigstens einen Infrarot-Strahler umfasst, wobei in der Abgasleitung eine Messeinrichtung angeordnet ist, die dazu ausgelegt ist, den Sauerstoffgehalt in dem durch das Beheizen des wenigstens einen Infrarot-Strahlers mit dem Biogas erzeugten Verbrennungsabgases zu messen. Besonders bevorzugt ist die Messeinrichtung eine Lambdasonde. Dadurch kann durch über den Sauerstoffgehalt in dem Abgas gesteuerte Zuführung von Verbrennungsgas zu dem Biogas die Zusammensetzung des zur Beheizung des bzw. der Infrarot-Strahler eingesetzten Biogases so eingestellt werden, dass eine gute Verbrennung des Biogases und somit gute Ausnutzung des Heizwertes des Biogases erreicht wird.

Aus diesem Grund ist es erfindungsgemäß auch vorgesehen, dass die Vorrichtung des Weiteren eine Regeleinheit umfasst, welche aufgrund des durch die Messeinrichtung in dem Verbrennungsabgas gemessenen Sauerstoffgehalts die Menge an dem Biogas vor dessen Zuführung in den wenigstens einen Infrarot-Strahler über die Zufuhrleitung zuzuführenden und zuzumischenden Verbrennungsgas regelt. Dadurch kann die Zusammensetzung des dem bzw. den Infrarot-Strahler(n) zugeführten Biogases so eingestellt werden, dass eine optimale Verbrennung des Biogases und somit optimale Ausnutzung des Heizwertes des Biogases erreicht wird.

In Weiterbildung des Erfindungsgedankens wird es vorgeschlagen, dass die Vorrichtung ferner eine Einrichtung zur Bestimmung des Heizwertes des Biogases vor dessen Zuführung in den wenigstens einen Infrarot-Strahler umfasst. Beispielsweise kann die Einrichtung zur Bestimmung des Heizwertes des Biogases einen Verbrennungskalorimeter umfassen.

Insbesondere ist es bevorzugt, dass in der Vorrichtung eine Regeleinheit enthalten ist, welche die Menge des pro Zeiteinheit dem wenigstens einen Infrarot-Strahler zuzuführenden Biogases sowie die Menge des pro Zeiteinheit dem Biogas zuzuführenden Verbrennungsgases so regelt, dass der wenigstens eine Infrarot-Strahler eine gleichbleibende Wärmeabstrahlleistung aufweist.

Zusätzlich kann die Vorrichtung eine Zufuhrleitung für Erdgas in die Leitung zur Zuführung von Biogas aus der Vorrichtung zur Erzeugung von Biogas zu wenigstens einem des wenigstens einen Infrarot-Strahlers aufweisen, um beispielsweise in dem Fall, dass aus produktionstechnischen Gründen zeitweise die Menge an in dem Verfahren erzeugten Biogas für eine ausreichende Beheizung der Infrarot-Strahler zu gering ist, dem Biogas vor dessen Zuführung in den wenigstens einen Infrarot-Strahler zur Beheizung desselben (geringe Mengen an) Erdgas zuzumischen.

Ferner kann die Vorrichtung eine Zufuhrleitung für Methan in die Leitung zur Zuführung von Biogas aus der Vorrichtung zur Erzeugung von Biogas zu wenigstens einen Infrarot-Strahlers aufweisen, um beispielsweise in dem Fall, dass aus produktionstechnischen Gründen zeitweise der Heizwert bzw. Methangehalt des in dem Verfahren erzeugten Biogases für eine ausreichende Beheizung der Infrarot-Strahler zu gering ist, dem Biogas vor dessen Zuführung in den wenigstens einen Infrarot-Strahler zur Beheizung desselben Methan zuzumischen.

Nachfolgend wird die vorliegende Erfindung unter Bezugnahme auf diese erläuternde, diese aber nicht einschränkende Figuren beschrieben.

Dabei zeigen die
- Fig. 1: schematisch eine Vorrichtung zum Herstellen einer gestrichenen Papierbahn gemäß einem Ausführungsbeispiel der vorliegenden Erfindung und
- Fig. 2: schematisch einen die Biogaszufuhrregelung zu der Trocknungseinrichutng im Detail zeigenden Ausschnitt der in der Fig. 1 gezeigten Vorrichtung.

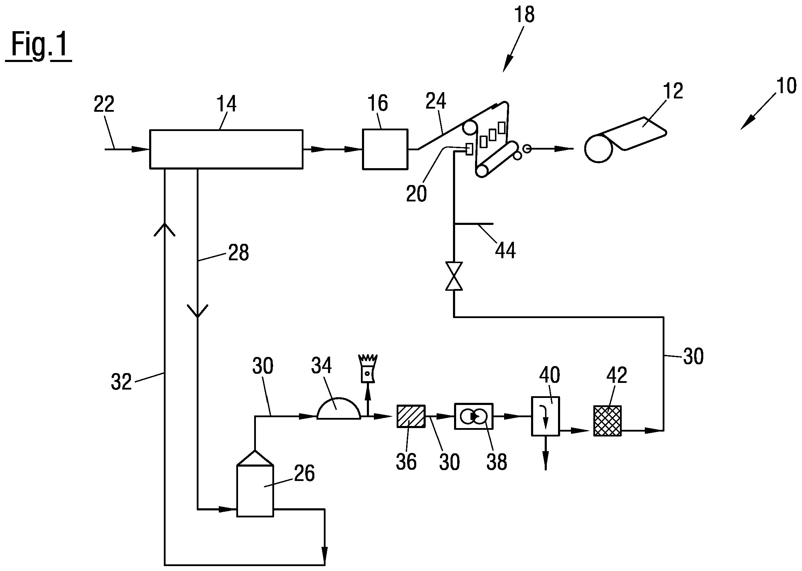

Die in der Fig. 1 gezeigte Vorrichtung 10 zum Herstellen einer gestrichenen Papierbahn 12 umfasst, in der Bahnlaufrichtung gesehen, eine Stoffaufbereitung 14, eine Streichmaschine 16 und eine Trocknungseinrichtung 18, wobei die Trocknungseinrichtung 18 mehrere Infrarot-Strahler 20 enthält, welche jeweils einen Teil einer durch die Trocknungseinrichtung 18 laufende Materialbahn trocknen. In die Stoffaufbereitung 14 mündet eine Zufuhrleitung für Altpapier 22, über welche der Stoffaufbereitung 14 bei dem Betrieb der Vorrichtung 10 kontinuierlich Altpapier zugeführt wird.

Aus dem Altpapier wird in der Stoffaufbereitung 14 nach Zuführung von Wasser durch Auflösen des Altpapiers in dem Wasser Faserstoff erzeugt wird, welcher dann zu der Materialbahn verfestigt und in der Streichmaschine 16 mit Strich beschichtet wird, bevor die so beschichtete Materialbahn 24 in der Trocknungseinrichtung 18 über die Infrarot-Strahler 20 getrocknet und damit zu der gestrichenen Papierbahn 12 verarbeitet wird.

Für den Betrieb der Stoffaufbereitung 14 werden erhebliche Mengen an Wasser benötigt, welches in der Stoffaufbereitung als Prozesswasser im Kreislauf geführt wird. Während des Betriebs der Stoffaufbereitung 14 wird das Prozesswasser mit aus dem Altpapier stammenden Substanzen, wie u.a. mit organischen Verbindungen, verunreinigt. Um das Prozesswasser zu reinigen, umfasst die Vorrichtung 10 einen anaerobe Mikroorganismen enthaltenden Reaktor 26, dem kontinuierlich ein Teil des Prozesswassers über die Prozesswasserzufuhrleitung 28 zugeführt wird. In dem Reaktor 26 wird das Prozesswasser mit den anaeroben Mikroorganismen kontaktiert, welche die in dem Prozesswasser als Verunreinigung enthaltenen organischen Verbindungen unter Bildung eines Methan und Kohlendioxid enthaltenden Biogases umsetzt. Das Biogas wird aus dem Reaktor 26 über die Biogasleitung 30 abgezogen, wohingegen das gereinigte Prozesswasser über die Prozesswasserrückfuhrleitung 32 in die Stoffaufbereitung 14 zurückgeführt wird.

Ferner umfasst die Vorrichtung 10 einen in der Biogasleitung 30 angeordneten Puffertank 34, in dem das Biogas zwischengespeichert wird, um eine kontinuierliche Zuführung einer ausreichenden Menge des Biogases zu den Infrarot-Strahlern 20 auch bei Schwankungen der Zusammensetzung des Biogases und/oder Schwankungen der erzeugten Menge des Biogases zu gewährleisten.

Außerdem umfasst die Vorrichtung 10 zur Konditionierung des Biogases in der Biogasleitung 30, in Strömungsrichtung gesehen, einen Gastrockner 36 zum Entfeuchten des Biogases, einen Kompressor 38 zum Verdichten des Biogases, eine Entschwefelungseinrichtung 40 zur Entfernung von schwefelhaltigen Verbindungen sowie eine Aktivkohle enthaltende Reinigungseinrichtung 42.

Des Weiteren umfasst die Vorrichtung 10 eine Zufuhrleitung für Verbrennungsgas 44, über welche dem Biogas Luft zugeführt wird, bevor die so hergestellte Gasmischung den Infrarot-Strahlern 20 zur Beheizung zugeführt wird.

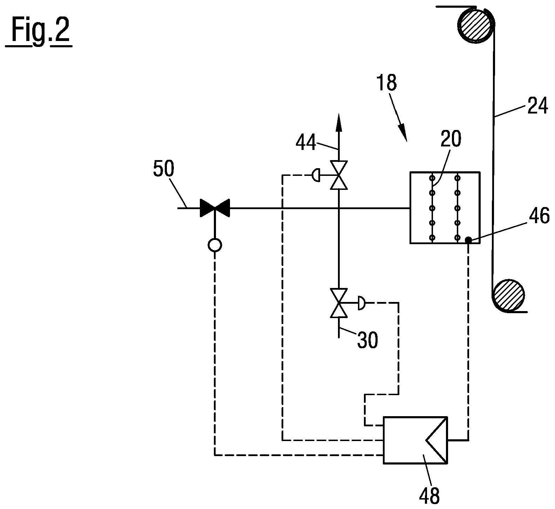

In der Fig. 2 ist schematisch ein die Biogaszufuhrregelung zu den Infrarot-Strahlern 20 im Detail zeigender Ausschnitt der in der Fig. 1 gezeigten Vorrichtung 10 dargestellt. Danach umfasst die Vorrichtung 10 eine Messeinrichtung 46, und zwar besonders bevorzugt eine Lambdasonde 46, über welche laufend der Sauerstoffgehalt in dem durch das Beheizen des wenigstens einen beheizten Teils 20 mit dem Biogas anfallenden Abgas gemessen wird. Zudem umfasst die Vorrichtung 10 eine Regeleinrichtung 48, welche aufgrund des durch die Messeinrichtung 46 in dem Verbrennungsabgas gemessenen Sauerstoffgehalts die Menge an dem Biogas vor dessen Zuführung in die Infrarot-Strahler 20 über die Zufuhrleitung für Verbrennungsgas 44 zuzuführenden und zuzumischenden Verbrennungsgas regelt. Dadurch wird die Zusammensetzung des zur Beheizung der Infrarot-Strahler 20 eingesetzten Biogases so eingestellt, dass eine optimale Verbrennung des Biogases in den Infrarot-Strahlern 20 und somit eine optimale Ausnutzung des Heizwertes des Biogases erreicht wird.

Zudem umfasst die Vorrichtung 10 eine Zufuhrleitung für Erdgas 50, welche in die Biogasleitung 30 mündet, um dem Biogas bei Bedarf über die Regeleinrichtung 48 geregelt Erdgas zuzuführen. Dies kann notwendig sein, wenn aus produktionstechnischen Gründen zeitweise die Menge an in dem Verfahren erzeugtem Biogas für eine ausreichende Beheizung der Infrarot-Strahler 20 zu gering ist.

### Bezugszeichenliste

- 10: Vorrichtung zum Herstellen einer gestrichenen Papierbahn
- 12: Gestrichene Papierbahn
- 14: Stoffaufbereitung
- 16: Streichmaschine
- 18: Trocknungseinrichtung
- 20: Infrarot-Strahler
- 22: Zufuhrleitung für Altpapier
- 24: Materialbahn
- 26: anaerobe Mikroorganismen enthaltender Reaktor
- 28: Prozesswasserzufuhrleitung
- 30: Biogasleitung
- 32: Prozesswasserrückfuhrleitung
- 34: Puffertank
- 36: Gastrockner
- 38: Kompressor
- 40: Entschwefelungseinrichtung
- 42: Reinigungseinrichtung
- 44: Zufuhrleitung für Verbrennungsgas
- 46: Messeinrichtung / Lambdasonde
- 48: Regeleinrichtung
- 50: Zufuhrleitung für Erdgas

## Patentansprüche

1. Verfahren zum Herstellen eines Gegenstandes, wobei das Verfahren das Trocknen des Gegenstandes (24, 12) oder eines Vorprodukts davon umfasst, bei dem der Gegenstand (24, 12) oder ein Vorprodukt davon in wenigstens einer Trocknungseinrichtung (18) zumindest teilweise getrocknet wird, wobei mindestens eine der wenigstens einen Trocknungseinheit (18) wenigstens einen zumindest teilweise mit Biogas beheizten Infrarot-Strahler (20) enthält, wobei dem Biogas vor dessen Zuführung in den wenigstens einen Infrarot-Strahler (20) zur Beheizung desselben ein aus der aus Sauerstoff, Luft oder anderen Sauerstoff enthaltenden Gasen bestehenden Gruppe ausgewähltes Verbrennungsgas zugemischt wird und in dem durch das Beheizen des wenigstens einen Infrarot-Strahlers (20) mit dem Biogas anfallenden Abgas mit einer Messeinrichtung (46) der Sauerstoffgehalt gemessen wird und aufgrund des gemessenen Sauerstoffgehalts die Menge an dem Biogas vor dessen Zuführung in den wenigstens einen Infrarot-Strahler (20) zugemischten Verbrennungsgas geregelt wird.

2. Verfahren nach Anspruch 1, wobei bei dem Verfahren auch Abwasser und/oder ein anderer Reststoff erzeugt wird, wobei das Biogas durch Behandeln des erzeugten Abwassers und/oder anderen Reststoffs hergestellt wird.

3. Verfahren nach Anspruch 1 oder 2, wobei das Biogas Methan enthält.

4. Verfahren nach zumindest einem der vorstehenden Ansprüche, wobei bei dem Verfahren Prozesswasser im Kreislauf geführt wird, welches bei dem Verfahren mit organischen Verbindungen verunreinigt wird, wobei das Biogas durch Behandeln des erzeugten Prozesswassers hergestellt wird.

5. Verfahren nach zumindest einem der vorstehenden Ansprüche, wobei mit dem Verfahren eine laufende Materialbahn (24, 12) hergestellt und diese in der wenigstens einen Trocknungseinrichtung (18) zumindest teilweise getrocknet wird, wobei bei dem Verfahren organische Verbindungen enthaltendes Abwasser anfällt und/oder Prozesswasser im Kreislauf geführt wird, welches bei dem Verfahren mit organischen Verbindungen verunreinigt wird, wobei das Biogas durch Behandeln des Abwassers und/oder Prozesswassers in einem anaerobe Mikroorganismen enthaltendem Reaktor (26) erzeugt wird, wobei die anaeroben Mikroorganismen in dem Abwasser und/oder Prozesswasser enthaltende organische Verbindungen zu dem Biogas umsetzen, wobei mit dem Verfahren vorzugsweise eine gestrichene Papier- oder Kartonbahn (12) hergestellt wird.

6. Verfahren nach zumindest einem der vorstehenden Ansprüche, wobei das Biogas vor dessen Verwendung zum Beheizen des wenigstens einen Infrarot-Strahlers (20) konditioniert wird, wobei das Biogas zur Konditionierung einem oder mehreren der folgenden Behandlungsschritte unterzogen wird:
i) Gastrocknung,
ii) Kompression,
iii) Entschwefelung,
iv) Reinigung mit Aktivkohle.

7. Verfahren nach zumindest einem der vorstehenden Ansprüche, wobei das Biogas vor dessen Verwendung zum Beheizen des wenigstens einen Infrarot-Strahlers (20) in einem Puffertank (34) zwischengespeichert wird, um eine kontinuierliche Zuführung einer ausreichenden Menge des Biogases zu dem wenigstens einen Infrarot-Strahler (20) auch bei Schwankungen der Zusammensetzung des Biogases und/oder Schwankungen der erzeugten Menge des Biogases gewährleisten zu können.

8. Verfahren nach zumindest einem der vorstehenden Ansprüche, wobei - die Messeinrichtung (46) eine Lambdasonde (46) ist.

9. Verfahren nach zumindest einem der vorstehenden Ansprüche, wobei der Heizwert des Biogases vor dessen Zuführung in den wenigstens einen Infrarot-Strahler (20) bestimmt wird, wobei aufgrund des bestimmten Heizwertes des Biogases die Menge des pro Zeiteinheit dem wenigstens einen Infrarot-Strahler (20) zuzuführenden Biogases geregelt wird.

10. Verfahren nach zumindest einem der vorstehenden Ansprüche, wobei die Menge des pro Zeiteinheit dem wenigstens einen Infrarot-Strahler (20) zuzuführenden Biogases sowie die Menge des pro Zeiteinheit dem Biogas zuzuführenden Verbrennungsgases so geregelt werden, dass der wenigstens eine Infrarot-Strahler (20) eine gleichbleibende Wärmeabstrahlleistung aufweist.

11. Vorrichtung (10) zur Durchführung eines Verfahrens zum Herstellen eines Gegenstandes nach zumindest einem der vorstehenden Ansprüche, wobe die Vorrichtung umfasst:
a) wenigstens eine Trocknungseinrichtung (18), wobei wenigstens eine Trocknungseinrichtung (18) ein oder mehrere Infrarot-Strahler (20) enthält,
b) eine Vorrichtung zur Erzeugung von Biogas (26) aus wenigstens einem ausgewählt aus der Gruppe bestehend aus Abwasser, Prozesswasser, anderen Reststoffen und beliebigen Kombinationen von zwei oder mehr der vorgenannten,
c) eine Leitung (30) zur Zuführung von Biogas aus der Vorrichtung zur Erzeugung von Biogas (26) zu wenigstens einer der wenigstens einen Trocknungseinrichtung (18) und
d) wenigstens eine Verbrennungsgaszufuhrleitung (44) zur Zumischung von einem aus der aus Sauerstoff, Luft oder anderen Sauerstoff enthaltenden Gasen bestehenden Gruppe ausgewählten Verbrennungsgas zu dem Biogas vor dessen Zuführung in die wenigstens eine Trocknungseinrichtung (18), wobei
die Vorrichtung (10) außerdem eine Abgasleitung zur Abführung des in der wenigstens einen Infrarot-Strahler (20) erzeugten Verbrennungsabgases aus dem wenigstens einen Infrarot-Strahler (20) umfasst, wobei in der Abgasleitung eine Messeinrichtung (46) angeordnet ist, die dazu ausgelegt ist, den Sauerstoffgehalt in dem durch das Beheizen des wenigstens einen Infrarot-Strahlers (20) mit dem Biogas anfallenden Abgases zu messen,
die Vorrichtung (10) des Weiteren eine Regeleinheit (48) umfasst, welche aufgrund des durch die Messeinrichtung (46) gemessenen Sauerstoffgehalts die Menge an dem Biogas vor dessen Zuführung in den wenigstens einen Infrarot-Strahler (20) über die Zufuhrleitung (44) zuzuführenden und zuzumischenden Verbrennungsgas regelt, und
die Leitung (30) zur Zuführung von Biogas aus der Vorrichtung zur Erzeugung von Biogas (26) zu wenigstens einem Infrarot-Strahler (20) ausgelegt ist.

12. Vorrichtung (10) nach Anspruch 11, wobei die Vorrichtung zur Erzeugung von Biogas (26) ein Reaktor (26) ist, der anaerobe Mikroorganismen enthält, welche organische Verbindungen zu einem Biogas umsetzen können.

13. Vorrichtung (10) nach Anspruch 11 oder 12, wobei die Vorrichtung (10) ferner wenigstens eine der folgenden Konditionierungseinrichtungen umfasst:
i) einen oder mehrere Gastrockner (36) zum Entfeuchten des Biogases,
ii) einen oder mehrere Kompressoren (38) zum Verdichten des Biogases,
iii) eine oder mehrere Entschwefelungseinrichtungen (40) zur Entfernung von schwefelhaltigen Verbindungen,
iv) eine oder mehrere Aktivkohle enthaltende Reinigungseinrichtungen (42).

14. Vorrichtung (10) nach zumindest einem der Ansprüche 11 bis 13, wobei die Vorrichtung (10) zudem einen in der Leitung (30) angeordneten Puffertank (34) umfasst, in dem Biogas vor dessen Verwendung zum Beheizen der wenigstens einen Infrarot-Strahlers (20) zwischengespeichert wird, um eine kontinuierliche Zuführung einer ausreichenden Menge des Biogases zu der wenigstens einen Infrarot-Strahler (20) auch bei Schwankungen der Zusammensetzung des Biogases und/oder Schwankungen der erzeugten Menge des Biogases gewährleisten zu können.

15. Vorrichtung (10) nach zumindest einem der Ansprüche 11 bis 14, wobei die Messeinrichtung (46) eine Lambdasonde (46) ist.

## Claims

1. Method for producing an object, wherein the method comprises drying the object (24, 12) or a primary product thereof, the object (24, 12) or a primary product thereof being at least partially dried in at least one drying device (18), wherein at least one of the at least one drying units (18) contains at least one infrared radiator (20) which is at least partially heated by means of biogas, wherein a combustion gas selected from the group consisting of oxygen, air or other oxygen-containing gases is added to the biogas before said biogas is supplied to the at least one infrared radiator (20) in order to heat said at least one infrared radiator, and the oxygen content in the waste gas resulting from the at least one infrared radiator (20) being heated by means of the biogas is measured by means of a measuring device (46) and the amount of combustion gas added to the biogas before said biogas is supplied to the at least one infrared radiator (20) is controlled on the basis of the measured oxygen content.

2. Method according to claim 1,
wherein
waste water and/or some other residual product is also generated in the method, the biogas being produced by treating the generated waste water and/or other residual product.

3. Method according to either claim 1 or claim 2,
wherein
the biogas contains methane.

4. Method according to at least one of the preceding claims,
wherein
process water is circulated during the method, which water becomes contaminated with organic compounds during the method, the biogas being produced by treating the generated process water.

5. Method according to at least one of the preceding claims,
wherein
a moving material web (24, 12) is produced by means of the method, and said web is at least partially dried in the at least one drying device (18), waste water containing organic compounds being produced and/or process water being circulated during the method, which process water becomes contaminated with organic compounds during the method, the biogas being generated by treating the waste water and/or process water in a reactor (26) containing anaerobic microorganisms, the anaerobic microorganisms converting organic compounds contained in the waster water and/or process water into the biogas, a coated paper or cardboard web (12) preferably being produced by means of the method.

6. Method according to at least one of the preceding claims,
wherein
the biogas is conditioned before being used to heat the at least one infrared radiator (20), the biogas being subjected to one or more of the following treatment steps for conditioning purposes:
i) gas drying,
ii) compression,
iii) desulfurization,
iv) cleaning with activated carbon.

7. Method according to at least one of the preceding claims,
wherein
the biogas is temporarily stored in a buffer tank (34) before being used to heat the at least one infrared radiator (20), so as to be able to ensure continuous supply of a sufficient amount of biogas to the at least one infrared radiator (20) even with fluctuations in the composition of the biogas and/or fluctuations in the amount of biogas generated.

8. Method according to at least one of the preceding claims,
wherein
the measuring device (46) is a lambda sensor (46).

9. Method according to at least one of the preceding claims,
wherein
the net calorific value of the biogas is determined before said biogas is supplied to the at least one infrared radiator (20), the amount of biogas to be supplied to the at least one infrared radiator (20) per unit of time being controlled on the basis of the determined net calorific value of the biogas.

10. Method according to at least one of the preceding claims,
wherein
the amount of biogas to be supplied to the at least one infrared radiator (20) per unit of time and the amount of combustion gas to be supplied to the biogas per unit of time are controlled such that the at least one infrared radiator (20) has a constant heat radiation output.

11. Apparatus (10) for carrying out a method for producing an object according to at least one of the preceding claims, wherein the apparatus comprises:
a) at least one drying device (18), wherein at least one drying device (18) contains one or more infrared radiators (20),
b) an apparatus (26) for generating biogas from at least one substance selected from the group consisting of waste water, process water, other residual products and any combination of two or more of these substances,
c) a line (30) for supplying biogas from the apparatus (26) for generating biogas to at least one of the at least one drying devices (18), and
d) at least one combustion gas supply line (44) for adding a combustion gas selected from the group consisting of oxygen, air or other oxygen-containing gases to the biogas before said biogas is supplied to the at least one drying device (18), wherein
the apparatus (10) also comprises a waste gas line for discharging the combustion waste gas generated in the at least one infrared radiator (20) from the at least one infrared radiator (20), wherein a measuring device (46) is arranged in the waste gas line, which measuring device is designed to measure the oxygen content in the combustion waste gas resulting from the at least one infrared radiator (20) being heated by means of the biogas, the apparatus (10) further comprises a control unit (48) which controls the amount of combustion gas to be supplied and added to the biogas via the supply line (44) before said biogas is supplied to the at least one infrared radiator (20) on the basis of the oxygen content measured by means of the measuring device (46), and
the line (30) is designed to supply biogas from the apparatus (26) for generating biogas to at least one infrared radiator (20).

12. Apparatus (10) according to claim 11,
wherein
the apparatus (26) for generating biogas is a reactor (26) which contains anaerobic microorganisms which can convert organic compounds into a biogas.

13. Apparatus (10) according to either claim 11 or claim 12,
wherein
the apparatus (10) further comprises at least one of the following conditioning devices:
i) one or more gas dryers (36) for removing moisture from the biogas,
ii) one or more compressors (38) for compressing the biogas,
iii) one or more desulphurization devices (40) for removing sulfur-containing compounds,
iv) one or more cleaning devices (42) containing activated carbon.

14. Apparatus (10) according to at least one of claims 11 to 13,
wherein
the apparatus (10) also comprises a buffer tank (34) arranged in the line (30), in which tank biogas is temporarily stored before being used to heat the at least one infrared radiator (20), so as to be able to ensure continuous supply of a sufficient amount of biogas to the at least one infrared radiator (20) even with fluctuations in the composition of the biogas and/or fluctuations in the amount of biogas generated.

15. Apparatus (10) according to at least one of claims 11 to 14,
wherein
the measuring device (46) is a lambda sensor (46).

## Revendications

1. Procédé de production d'un objet, dans lequel le procédé comprend le séchage de l'objet (24, 12) ou d'un produit préliminaire de celui-ci, dans lequel l'objet (24, 12) ou un produit préliminaire de celui-ci est au moins partiellement séché dans au moins un appareil de séchage (18), au moins l'un de l'au moins un appareil de séchage (18) contenant au moins un émetteur infrarouge (20) qui est au moins partiellement chauffé avec du biogaz, le biogaz, avant d'être alimenté à l'au moins un émetteur infrarouge (20) pour le chauffer, étant mélangé avec un gaz de combustion choisi dans le groupe constitué par l'oxygène, l'air ou d'autres gaz contenant de l'oxygène, et dans lequel la teneur en oxygène est mesurée avec un appareil de mesure (46) en chauffant l'au moins un émetteur infrarouge (20) avec le gaz d'échappement résultant du biogaz et, sur la base de la teneur en oxygène mesurée, la quantité de biogaz mélangé avec le gaz de combustion avant d'être alimenté à l'au moins un émetteur infrarouge (20) étant régulée.

2. Procédé selon la revendication 1,
dans lequel
dans le procédé, des eaux usées et/ou d'autres résidus sont également générés, le biogaz étant produit en traitant les eaux usées et/ou les autres résidus générés.

3. Procédé selon la revendication 1 ou 2,
dans lequel
le biogaz contient du méthane.

4. Procédé selon au moins l'une des revendications précédentes,
dans lequel
de l'eau de processus est mise en circulation dans le procédé, laquelle est contaminée par des composés organiques dans le procédé, le biogaz étant produit en traitant l'eau de processus générée.

5. Procédé selon au moins l'une des revendications précédentes,
dans lequel
une bande de matériau courante (24, 12) est produite avec le procédé et celle-ci est au moins partiellement séchée dans l'au moins un appareil de séchage (18), des eaux usées contenant des composés organiques résultant du précédé et/ou de l'eau de processus étant mise en circulation, laquelle étant contaminée par des composés organiques dans le procédé, le biogaz étant généré en traitant les eaux usées et/ou l'eau de processus dans un réacteur (26) contenant des micro-organismes anaérobies, les micro-organismes anaérobies convertissant les composés organiques contenus dans les eaux usées et/ou l'eau de processus en biogaz, une bande de papier ou de carton couchée (12) étant de préférence produite avec le procédé.

6. Procédé selon au moins l'une des revendications précédentes, dans lequel
le biogaz est conditionné avant d'être utilisé pour chauffer l'au moins un émetteur infrarouge (20), le biogaz étant soumis à une ou plusieurs des étapes de traitement suivantes pour le conditionnement :
i) séchage du gaz,
ii) compression,
iii) désulfuration,
iv) nettoyage au charbon actif.

7. Procédé selon au moins l'une des revendications précédentes, dans lequel
le biogaz est temporairement stocké dans un réservoir tampon (34) avant d'être utilisé pour chauffer l'au moins un émetteur infrarouge (20) afin d'assurer une alimentation continue d'une quantité suffisante de biogaz à l'au moins un émetteur infrarouge (20), même lors de fluctuations de la composition du biogaz et/ou de fluctuations de la quantité de biogaz générée.

8. Procédé selon au moins l'une des revendications précédentes,
dans lequel
l'appareil de mesure (46) est une sonde lambda (46).

9. Procédé selon au moins l'une des revendications précédentes,
dans lequel
le pouvoir calorifique du biogaz est déterminé avant qu'il ne soit alimenté à l'au moins un émetteur infrarouge (20), la quantité de biogaz à alimenter à l'au moins un émetteur infrarouge (20) par unité de temps étant régulée sur la base du pouvoir calorifique déterminé du biogaz.

10. Procédé selon au moins l'une des revendications précédentes,
dans lequel
la quantité de biogaz à alimenter à l'au moins un émetteur infrarouge (20) par unité de temps ainsi que la quantité de gaz de combustion à alimenter au biogaz par unité de temps peuvent être régulées de telle sorte que l'au moins un émetteur infrarouge (20) présente une puissance de rayonnement thermique constante.

11. Dispositif (10) permettant la mise en œuvre d'un procédé pour la production d'un objet selon au moins l'une des revendications précédentes, dans lequel le dispositif comprend :
a) au moins un appareil de séchage (18), au moins un appareil de séchage (18) contenant un ou plusieurs émetteurs infrarouges (20),
b) un dispositif permettant de générer du biogaz (26) à partir d'au moins un élément sélectionné dans le groupe constitué par les eaux usées, l'eau de processus, d'autres résidus et toute combinaison de deux ou plus des éléments précédents,
c) une conduite (30) permettant d'alimenter du biogaz provenant du dispositif permettant de générer du biogaz (26) à au moins l'un de l'au moins un appareil de séchage (18) et
d) au moins une conduite d'alimentation en gaz de combustion (44) permettant de mélanger un gaz de combustion sélectionné dans le groupe constitué par l'oxygène, l'air ou d'autres gaz contenant de l'oxygène au biogaz avant qu'il ne soit alimenté à l'au moins un appareil de séchage (18),
le dispositif (10) comprenant également une conduite de gaz d'échappement permettant d'évacuer les gaz d'échappement de combustion générés dans l'au moins un émetteur infrarouge (20) depuis l'au moins un émetteur infrarouge (20), un appareil de mesure (46) étant disposé dans la conduite de gaz d'échappement et configuré pour mesurer la teneur en oxygène dans les gaz d'échappement résultant du chauffage de l'au moins un émetteur infrarouge (20) avec le biogaz,
le dispositif (10) comprenant en outre une unité de commande (48) qui, sur la base de la teneur en oxygène mesurée par l'appareil de mesure (46), régule la quantité de gaz de combustion à alimenter au biogaz par l'intermédiaire de la conduite d'alimentation (44) et à mélanger avec celui-ci avant qu'il ne soit alimenté à l'au moins un émetteur infrarouge (20), et
la conduite (30) est conçue pour alimenter du biogaz provenant du dispositif permettant de générer du biogaz (26) à au moins un émetteur infrarouge (20).

12. Dispositif (10) selon la revendication 11,
dans lequel
le dispositif permettant de générer du biogaz (26) est un réacteur (26) contenant des micro-organismes anaérobies capables de transformer des composés organiques en biogaz.

13. Dispositif (10) selon la revendication 11 ou 12,
dans lequel
le dispositif (10) comprend en outre au moins l'un des appareils de conditionnement suivants :
i) un ou plusieurs sécheurs de gaz (36) permettant de déshumidifier le biogaz,
ii) un ou plusieurs compresseurs (38) permettant de comprimer le biogaz,
iii) un ou plusieurs dispositifs de désulfuration (40) permettant d'éliminer les composés contenant du soufre,
iv) un ou plusieurs dispositifs de nettoyage (42) contenant du charbon actif.

14. Dispositif (10) selon au moins l'une des revendications 11 à 13,
dans lequel
le dispositif (10) comprend également un réservoir tampon (34) disposé dans la conduite (30), dans lequel du biogaz est temporairement stocké avant qu'il ne soit utilisé pour chauffer l'au moins un émetteur infrarouge (20), afin d'assurer une alimentation continue d'une quantité suffisante de biogaz à l'au moins un émetteur infrarouge (20), même lors de fluctuations de la composition du biogaz et/ou de fluctuations de la quantité de biogaz générée.

15. Dispositif (10) selon au moins l'une des revendications 11 à 14, dans lequel
l'appareil de mesure (46) est une sonde lambda (46).
